(19) Europäisches Patentamt

European Patent Office

Office européen des brevets

(11) **EP 1 424 319 B1**

(12) **EUROPÄISCHE PATENTSCHRIFT**

(45) Veröffentlichungstag und Bekanntmachung des Hinweises auf die Patenterteilung:
**06.07.2005 Patentblatt 2005/27**

(51) Int Cl.⁷: **C07C 31/18**, B01J 2/04,
B01D 1/18, A61K 9/20

(21) Anmeldenummer: **02023519.8**

(22) Anmeldetag: **22.10.2002**

(54) **Sprühgetrockneter Xylitol und Verfahren zu dessen Herstellung**

Spray-dried xylitol and process for its production

Xylitol séchée par pulvérisation et procédé de sa préparation

(84) Benannte Vertragsstaaten:
**AT BE BG CH CY CZ DE DK EE ES FI FR GB GR IE IT LI LU MC NL PT SE SK TR**

(43) Veröffentlichungstag der Anmeldung:
**02.06.2004 Patentblatt 2004/23**

(73) Patentinhaber: **DHW Deutsche Hydrierwerke GmbH Rodleben**
**06862 Rodleben (DE)**

(72) Erfinder:
• **Ballnus, Martina**
**06862 Rodleben (DE)**
• **Dichte, Burkhard**
**39261 Zerbst (DE)**
• **Knofe, Eberhard**
**06847 Dessau (DE)**
• **Konetzke, Gerhard**
**06847 Dessau (DE)**
• **Stanneveld, Detlev**
**06862 Rosslau (DE)**
• **Szilagyi, Zoltan**
**61184 Karmen (DE)**
• **Weidemann, Frank**
**06862 Meinsdorf (DE)**

(74) Vertreter: **Tragsdorf, Bodo, Dipl.-Ing.**
**Patentanwalt**
**Heinrich-Heine-Strasse 3**
**06844 Dessau (DE)**

(56) Entgegenhaltungen:
WO-A-99/59426          DE-A- 19 845 339
US-A- 5 204 115        US-A- 5 958 471

## Beschreibung

Die Erfindung betrifft einen sprühgetrockneten Xylitol, bestehend aus Xylitolpartikeln hoher Reinheit mit einem Xylitolgehalt von mindestens 98 Gew.-%, und ein Verfahren zu dessen Herstellung.

Es ist allgemein bekannt, Xylitol als Zuckeraustausch- und Trägerstoff in pharmazeutischen Präparaten und Komprimaten in der Lebensmittelindustrie, insbesondere in Form von Lutsch- oder Kau-Tabletten, einzusetzen. Bisher bekannter Rein-Xylitol besitzt jedoch unabhängig von der Art der Herstellung, sehr schlechte Tablettiereigenschaften. Um diese zu verbessern ist es bereits bekannt (DE 198 45 339 A1), Xylitol mit einem Xylitolgehalt von größer als 90 Gew.-%, bezogen auf den Gesamtpolyolgehalt, zusammen mit einem weiteren Polyol, in Wasser gelöst, durch eine sogenannte Co-Sprühtrocknung herzustellen. Diese erfolgt durch Versprühen des Gemisches in einem Luftstrom bei einer Temperatur von 120 bis 300 °C, Verwirbeln des Gemisches in einem Luftstrom bei einer Temperatur von 30 bis 110 °C unter Verdampfung des Wassers und abschließender Isolierung des erhaltenen Hilfsstoffes, der direkt zu Tabletten verpressbar ist. In dieser Druckschrift sind als Vergleich auch die Eigenschaften von Tabletten aus Xylitol, der durch Sprühgranulierung in der Wirbelschicht gewonnen wurde, angegeben. Die Ergebnisse zeigen, dass der sprühgranulierte Rein-Xylitol für eine direkte Tablettierung ohne weitere Zusätze ungeeignet ist. Für die hergestellten Tabletten sind bei einem Pressdruck von 20 kN die Tablettenhärten von ca. 60 N zu gering und der Abrieb zu hoch. Außerdem wird durch den Zusatz eines weiteren Polyols die vorteilhafte antikariogene Wirkung des reinen Xylitols verringert. Ein Zusatz von Sorbit zu Xylitol wirkt sich aufgrund der hygroskopischen Eigenschaft des Sorbits nachteilig auf die Tablettenqualität aus.

Seitens der Anwender werden in der Regel Tablettenhärten von mindestens 140 kN gefordert. Derart hohe Tablettenhärten werden in der Praxis bisher nur bei sprühgranuliertem Xylitol durch den Zusatz von Bindemitteln, wie zum Beispiel CMC (Carboxymethylcellulose) in Einsatzmengen von ca. 2 %, erzielt. Das in den Tabletten enthaltene CMC wirkt sich jedoch nachteilig auf die anwendungstechnischen Eigenschaften, insbesondere die Sensorik aus.

In der WO 99/59426 ist ein kristallines Xylitol-Produkt beschrieben, das aus einer Vielzahl an suspensionskristallisierten Mikrokristallen mit einer Größe von kleiner als 50 μ besteht, die in einer zufälligen Auswahl, zu Partikeln mit einer Größe von 0,1 bis 10 mm, agglomeriert sind. Zur Herstellung der Xylitolpartikel wird eine hochreine Xylitollösung mit einem Xylitolgehalt von 30 bis 80 Gew.-% eingesetzt. Diese wird in einen beheizten Tank eingeleitet und bei einer Temperatur von 45 bis 80 °C unter hohem Druck, in einem heißen Gas suspendiert und versprüht, unter gleichzeitiger Zuführung von im Kreislauf geführten mikrokristallinen Xylitolpartikeln. Auf einem am Boden des Sprühturmes angeordneten beheizten Sieb bilden sich Agglomerate in einer Größe von 0,5 bis 5 cm. Die aus den Agglomeraten bestehende Xylitolschicht muss während einer relativ langen Zeitdauer, von z.B. einer Stunde, konditioniert und danach noch fein gemahlen und gesiebt werden. Eine Teilmenge der erhaltenen gesiebten Partikel wird wieder als Kreislaufprodukt eingesetzt. Die vorgeschlagenen Verfahrensbedingungen erfordern den Einsatz eines integrierten Fließbettes, da der Agglomerierungs- und Trocknungsprozess innerhalb des Sprühturmes noch nicht abgeschlossen ist.

Dieser Herstellungsprozess ist sehr aufwendig und erfordert außerdem eine spezielle Mikrokristallisationsapparatur sowie gesonderte Sprühdüsen zur Bildung von extrem kleinen Tropfen aus denen die Mikrokristalle entstehen sollen. Dabei muss verfahrenstechnisch sichergestellt werden, dass sich die einzelnen Tropfen nicht berühren können, bevor diese in ausreichendem Maße verfestigt sind.

Aus dem Xylitol hergestellte Tabletten einer Dicke von ca. 4,1 mm, unter Zusatz von 0,5 % Magnesiumstearat, weisen eine Härte von 43 bis 70 N auf.

Die für diesen Xylitol angegebenen physikalischen Eigenschaften und die erzielbare Tablettenhärte sind für die meisten Weiterverarbeitungen und Anwendungen in der Lebensmittel- und pharmazeutischen Industrie nicht ausreichend.

Der Erfindung liegt die Aufgabe zugrunde, einen pulverförmigen Xylitol bereitzustellen, der sich durch verbesserte anwendungstechnische Eigenschaften, insbesondere für die Verpressbarkeit zu Tabletten, auszeichnet. Außerdem soll ein geeignetes kostengünstiges Verfahren zur Herstellung von pulverförmigem Xylitol geschaffen werden.

Erfindungsgemäß wird die Aufgabe durch die im Anspruch 1 angegebenen Merkmale gelöst. Geeignete Ausgestaltungen und Weiterbildungen sind in den Ansprüchen 2 und 3 angegeben. Ein geeignetes Herstellungsverfahren ist Gegenstand des Anspruches 4. Die Ansprüche 5 bis 7 beziehen sich auf weitere Ausgestaltungen der Verfahrensweise. Gegenstand der Ansprüche 8 und 9 sind bestimmte Verwendungen und die Ansprüche 10 bis 12 betreffen bindemittelfreie Komprimate.

Der vorgeschlagene sprühgetrocknete Xylitol mit einem Xylitolgehalt von mindestens 98 Gew.-% zeichnet sich durch eine ganz spezielle Partikelmorphologie aus, die zu einem Produkt mit hervorragenden anwendungstechnischen Eigenschaften führt. Das bereits am Sprühturmaustritt anfallende Rein-Xylitolpulver besitzt ein Kornspektrum von ca. 0,1 bis 0,8 mm, vorzugsweise mit einem Kornanteil von 0,1 bis 0,315 mm von größer als 50 %. Die einzelnen Xylitolpartikel weisen eine unregelmäßige, zerklüftete Oberfläche auf und fallen als rieselfähiges Produkt mit einem Wassergehalt von ca. 0,2 bis 1 % an, mit einer Schüttdichte, bestimmt nach DIN ISO 3944, von kleiner als 0,65 kg/l, einer Lösezeit von kleiner als 10 s und einer Rieselzeit von kleiner als 12 s/100 ml oder einem Schüttwinkel von kleiner als 33°.

Das Xylitolpulver zeichnet sich durch wesentlich verbesserte Tablettiereigenschaften, insbesondere bei der Direkttablettierung, aus. Bereits bei Pressdrücken von ca. 10 bis 15 kN werden Tabletten mit einer Bruchfestigkeit von mindestens 140 N und einer Biegefestigkeit von mindestens 10 N/mm$^2$, bestimmt nach einer Mindestlagerzeit von 24 h, erhalten. Wesentlich ist dabei, dass das Xylitolpulver keine Zusätze enthält, also ein Rein-Xylitolpulver ist, und die Herstellung der Tabletten ohne Zusatz von Bindemittel erfolgt. Aufgrund der bindmittelfreien Herstellung und der speziellen Partikelmorphologie des Xylitolpulvers besitzen die hergestellten Komprimate ein gleichmäßigeres Aussehen und verbesserte sensorische Eigenschaften. Die Tabletten weisen eine glatte Oberfläche auf, die während des Lutschen erhalten bleibt und sich nicht nach und nach aufraut. Erstmals können aus reinem Xylitol auch ohne Zusatz von Bindemitteln wie z. B. CMC, anderer Polyole oder entsprechender Hilfsmittel bei der Versprühung, Tabletten mit der geforderten hohen Härte hergestellt werden. Durch die unregelmäßige raue Oberfläche ist der erfindungsgemäße Xylitol zusätzlich in der Lage, Zusatzstoffe, wie z. B. Aromastoffe, Farbstoffe, Wirkstoffe, Vitamine und Spurenelemente, ausgezeichnet zu binden. Die Aufnahmekapazität liegt wesentlich höher als bei den bisher bekannten Xylitolen. Es gelingt außerdem, mit diesen Zusatzstoffen wesentlich homogenere Mischungen herzustellen. Das Rein-Xylitolpulver ist sehr gut zur Herstellung von antikariogenem Kaugummi geeignet. Bindemittelfreie Komprimate bestehen aus Rein-Xylitolpulver mit einem Kornspektrum von ca. 0,1 bis 0,8 mm, einem Wassergehalt von < 0,5 %, einer Schüttdichte, bestimmt nach DIN ISO 3944, von kleiner als 0,65 kg/l, einer Lösezeit von kleiner als 10 s und einer Rieselzeit von kleiner als 12 s/100 ml oder einem Schüttwinkel von kleiner als 33°, wobei die einzelnen Xylitolpartikel eine unregelmäßige, zerklüftete Oberfläche besitzen. Gegebenenfalls können die Komprimate auch unter Verwendung eines an sich bekannten Gleitmittels hergestellt sein und noch einen oder mehrere der vorgenannten Zusatzstoffe enthalten.

Durch die sehr gute Rieselfähigkeit des Xylitolpulvers verbessern sich die Lagereigenschaften grundlegend. Auch in größeren Gebinden abgepackt verklumpt das Produkt nach längerer Lagerzeit nicht und kann problemlos mittels herkömmlichen Fördereinrichtungen transportiert werden.

Zur Herstellung des erfindungsgemäßen Xylitolpulvers wird folgende Verfahrensweise vorgeschlagen:

Die Xylitollösung mit einem Feststoffgehalt von 40 bis 80 Gew.-% wird auf eine Temperatur von 40 bis 95 °C erwärmt und in definierter Menge mittels eines Rotationszerstäubers, der mit Drehzahlen von 5500 bis 15000 U/min betrieben wird, in einem Sprühturm versprüht, unter gleichzeitiger Zuführung von im Kreislauf geführten pulverförmigem Xylitol in der 1 bis 3fachen Menge, bezogen auf die eingesetzte Menge an Xylitollösung. Vorzugsweise kann als Xylitollösung das bei der Hydrierung von Xylose anfallende Hydrierprodukt nach Abtrennen des Katalysators, Entsalzung und Eindampfung eingesetzt werden.

Dadurch entfallen aufwendige Kristallisationsprozesse und das anschließende Auflösen zur Herstellung der Versprühlösung.

In den Sprühturm wird gleichzeitig mit der Xylitollösung heiße Luft mit einer Temperatur von 110 bis 160 °C in einer Menge von 10 bis 16 Nm$^3$/kg Xylitollösung eingeblasen, wobei die Temperatur der eingeblasenen heißen Luft so geregelt wird, dass sich am Austritt des Sprühturmes eine nahezu konstante Lufttemperatur von 55 bis 60 °C einstellt. Dadurch wird am Sprühturmaustritt bereits rieselfähiges, direkt weiterverarbeitbares Pulver mit einem Kornspektrum von ca. 0,1 bis 0,8 mm mit einer unregelmäßig zerklüfteten Oberfläche und einem Wassergehalt von ca. 0,2 bis 1 % erhalten. Auf eine weitere zeit- und kostenaufwendige Aufarbeitung in einem nachgeschalteten Fließbett, wie dies bei anfallenden Agglomeraten der Fall ist, kann somit verzichtet werden. Das am Sprühturmaustritt anfallende Xylitolpulver besitzt bereits Schüttdichten von 0,5 bis 0,65 kg/l und eine ausgezeichnete Rieselfähigkeit.

Das für die anwendungstechnischen Eigenschaften vorteilhafte Korngrößenspektrum wird schon im Sprühturm gebildet. Damit entfallen ansonsten notwendige, aufwendige Mahl- und Siebprozesse. Durch die vorgeschlagenen verfahrenstechnischen Maßnahmen ist es gelungen, bereits am Sprühturmaustritt ein rieselfähiges Produkt mit einem vergleichsweise sehr niedrigen Wassergehalt von vorzugsweise 0,2 bis 0,3 % zu erhalten.

Sofern es erforderlich sein sollte, ein Produkt mit einem noch kleineren Wassergehalt zu erhalten, so ist es zweckmäßig, wenn das am Austritt des Sprühturmes anfallende Xylitolpulver auf einem nachgeschalteten Fließbett mittels Warmluft getrocknet und abschließend mittels entfeuchteter Kaltluft auf eine Temperatur von 40 bis 20 °C abgekühlt wird. Auf diese Weise lassen sich Wassergehalte von kleiner als 0,2 % erzielen.

Um die gewünschte Partikelmorphologie zu erreichen, müssen während des Sprühvorganges die Verfahrensparameter Xylitollösungstemperatur und -menge, Luftmenge und Lufttemperatur aufeinander abgestimmt werden, derart, dass sich am Austritt des Sprühturmes eine nahezu konstante Lufttemperatur von 55 bis 60 °C einstellt.

Versuche haben gezeigt, dass Lufttemperaturen außerhalb des vorgenannten Bereiches zu einem Produkt mit deutlich schlechteren Eigenschaftswerten führen.

Die Versprühung wird durch Zerstäuben mittels eines Rotationszerstäubers in einem auf eine Temperatur von 110 bis 160 °C, vorzugsweise 140 °C, erwärmten trockenen Luftstrom, der in einer Menge von 10 bis 16 Nm$^3$ pro kg Produkt zugeführt wird, durchgeführt. Der Feststoffgehalt der zur Versprühung eingesetzten Lösung wird auf 40 bis 80 %, vorzugsweise ca. 65 %, eingestellt. Die Xylitolpartikel, die sich bei der Entwässerung der Xylitollösungströpfchen ausbilden, erwärmen sich und trocknen spontan unter Bildung einer unregelmäßigen, zerklüfteten Oberfläche.

Die Verweilzeiten der Xylitolpartikel im Sprühturm betragen ca. 1 bis 2 min.

Unter den angegebenen Verfahrensbedingungen entsteht ein rieselfähiges Produkt in einem sehr engen Kornspektrum bereits im Sprühturm.

Der zur Versprühung benötigte Feststoffanteil in einer Menge von 1000 bis 3000 kg/h kann direkt am Sprühturmaustritt ausgekreist und am Sprühturmkopf in diesen zurückgeführt werden. Bei den bekannten Verfahrensweisen ist es stets erforderlich, dass das am Sprühturmaustritt anfallende Produkt erst nachbehandelt und gemahlen werden muss.

Das hergestellte Rein-Xylitolpulver ist insbesondere für den Einsatz in Lebensmitteln und Süßwaren, wie z.B. Kaugummiformulierungen, pharmazeutischen Präparaten und technischen Anwendungen bestimmt. Von besonderem Vorteil sind die hervorragenden Herstellungs- und anwendungstechnischen Eigenschaften zur Bildung von Komprimaten, wie zum Beispiel Tabletten.

Die Erfindung wird nachfolgend an einigen Beispielen erläutert.

A: Herstellung von pulverförmigem Xylitol

Die Sprühtrocknung einer unmittelbar nach der Hydrierung von Xylose anfallenden Hydrierlösung erfolgt im Sprühturm einer herkömmlichen Sprühtrocknungsanlage mit einem Rotationszerstäuber, wobei die Versuche gemäß Beispiel 1 und 2 in einer großtechnischen Anlage und der Versuch gemäß Beispiel 3 in einer Pilotanlage durchgeführt wurden.

Die Hydrier- bzw. Xylitollösung wird auf eine Konzentration von ca. 40 bis 70 Gew.-% eingeengt, in einem Wärmetauscher auf eine Temperatur von ca. 70 bis 90 °C erwärmt und dem im Kopfbereich des Sprühturmes angeordnetem Rotationszerstäuber, der mit einer Drehzahl von ca. 10000 bis 15000 U/min arbeitet, in einer Menge von 1250 bis 1300 kg/h (Beispiele 1 und 2) bzw. 1,2 kg/h (Beispiel 3) zugeführt. Am Kopf des Sprühturmes wird gleichzeitig die zur Sprühtrocknung benötigte, auf eine Temperatur von ca. 115 °C bis 130 °C erwärmte Luft in einer Menge von 12 bis 15 Nm$^3$/kg Xylitollösung eingeblasen. Gleichzeitig wird am Kopf des Sprühturmes das für die Sprühtrocknung benötigte Xylitolpulver, im Kreislauf, in einer Menge von 2500 bis 3600 kg/h (Beispiele 1 und 2) bzw. 1,2 kg/h (Beispiel 3) zugeführt, das direkt am Sprühturmaustritt ausgekreist wird. Von wesentlicher Bedeutung für die Verfahrensdurchführung ist, dass die Temperatur der zugeführten erwärmten Luft über eine Regelstrecke so eingestellt wird, dass am Sprühturmaustritt eine nahezu konstante Luftaustrittstemperatur von ca. 55 bis 60 °C herrscht.

Xylitolpulver wurde unter Einhaltung folgender Verfahrensparameter hergestellt:

| Verfahrensparameter | Beispiele | | |
|---|---|---|---|
| | 1 | 2 | 3 |
| Konz. der Xylitollösung (%) | 65 | 70 | 40 |
| Temp. der Xylitollösung (°C) | 90 | 85 | 70 |
| Menge der Xylitollösung (kg/h) | 1300 | 1250 | 1,2 |
| Drehzahl des Zerstäubers (U/min) | 15000 | 10000 | 13000 |
| Heißlufttemperatur (°C) | 120 ± 5 | 130 ± 5 | 115 ± 2 |
| Heißluftmenge (Nm$^3$/kg) | 12 | 15 | 14 |
| Xylitolpulvermenge (kg/h) | 3600 | 2500 | 1,2 |
| Luftaustrittstemperatur (°C) | 57 | 55 | 60 |

Unter vorgenannten Verfahrensbedingungen beträgt die Verweilzeit des Xylitols im Sprühturm ca. 1 min. Das am Sprühturmaustritt anfallende Xylitolpulver, das nicht im Kreislauf wieder in den Sprühturm zurückgeführt wird, kann entweder direkt weiterverarbeitet oder zwischengelagert werden. Zur weiteren Zwischenlagerung ist es zweckmäßig, den sprühgetrockneten Xylitol auf einem Fließbett mit Warmluft bis zu einem Wassergehalt von < 0,15 % zu trocknen und anschließend mit entfeuchteter Kaltluft auf eine Temperatur von ca. 40 bis 20 °C abzukühlen.

B: Eigenschaften des hergestellten Xylitolpulvers

An den nach den angegebenen Verfahrensparametern hergestellten Xylitolpulvern, die am Sprühturmaustritt entnommen wurden, wurden folgende Eigenschaftswerte ermittelt:

Schüttdichte gemäß DIN ISO 3944;
Wassergehalt: Bestimmung nach Karl Fischer;
Fließfähigkeit/Rieselzeit:
Bestimmung erfolgt entsprechend Europäische Pharmakopoe (Ph. Eur.), Pkt 2.9.16, verwendeter Trichter: Fordbecher mit Auslaufdüse (8mm);

Schüttwinkel $\alpha$: tan$\alpha$= Schütthöhe/Radius;

Lösezeit: Lösezeit von 1g in 200ml destilliertem Wasser bei 30°C unter Rühren.

Die ermittelten Eigenschaftswerte sind in der nachfolgenden Tabelle angegeben:

| Eigenschaften | Xylitolpulver gemäß den Beispielen | | | |
|---|---|---|---|---|
| | 1 | 2 | 3 | V |
| Schüttdichte (kg/l) | 0,61 | 0,62 | 0,61 | 0,81 |
| Schüttwinkel (°) | 28,9 | 31,3 | 29,8 | 41,2 |
| Wassergehalt (%) | 0,30 | 0,25 | 0,20 | 0,05 [1] |
| Rieselzeit (s/100 ml) | 10,56 | 11,02 | 10,43 | [2] |
| Lösezeit (s) | 6,6 | 5,8 | 7,1 | 12,02 |

[1] es handelt sich um ein nachgetrocknetes Produkt;

[2] nicht rieselfähig;

Das Vergleichsbeispiel V bezieht sich auf ein handelsübliches Xylitol-Produkt (Handelsname "Xylitol C"), dessen Eigenschaftswerte zu Vergleichszwecken bestimmt wurden.

Die vorstehende Tabelle zeigt, dass die aus dem erfindungsgemäßen sprühgetrockneten Rein-Xylitol hergestellten Tabletten aufgrund ihrer speziellen Partikelmorphologie, insbesondere der unregelmäßig zerklüfteten Oberfläche, ausgezeichnete Instanteigenschaften besitzen. Dies belegen die ermittelten niedrigen Lösezeiten von 5,8 bis 7,1 s, sowie ein deutlich verbessertes Rieselverhalten mit ca. 11 s. Dadurch bedingt ergibt sich auch ein erheblich niedriger Schüttwinkel gegenüber dem Vergleichsprodukt. Die Schüttgewichte mit ca. 0,6 kg/l sind ebenfalls sehr niedrig.

Diese hervorragenden Eigenschaften ermöglichen eine direkte Verpressbarkeit des Xylitolpulvers zu Komprimaten, insbesondere Tabletten.

An den Xylitol-Produkten gemäß den Beispielen 1 bis 3, die unmittelbar am Sprühturmaustritt entnommen wurden, und dem Vergleichsbeispiel V wurde das Kornspektrum nach dem Alpine Maschensiebverfahren bestimmt, wobei folgende Werte ermittelt wurden:

| Kornspektrum (%) | Xylitolpulver gemäß den Beispielen | | | |
|---|---|---|---|---|
| | 1 | 2 | 3 | V |
| 0,04 bis 1 mm | 3,4 | 0,9 | 0,9 | 1,0 |
| 0,1 bis 0,2 mm | 25,5 | 29,7 | 20,9 | 6,0 |
| 0,2 bis 0,315 mm | 53,9 | 48,5 | 41,9 | 25,5 |
| 0,315 bis 0,5 mm | 9,7 | 15,8 | 20,4 | 45,5 |
| 0,5 bis 0,8 mm | 0,7 | 52,7 | 8,9 | 20,0 |
| > 0,8 mm | 0 | 2,4 | 7 | 2,0 |

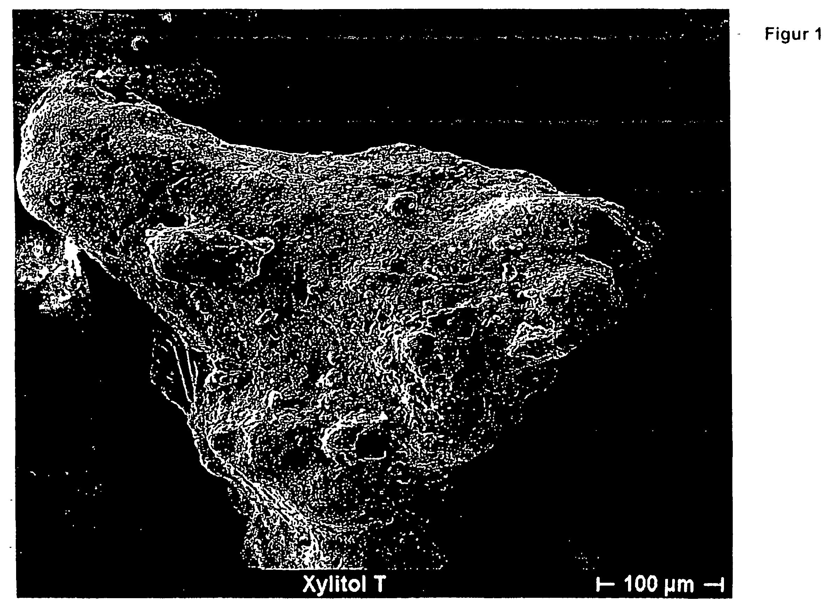

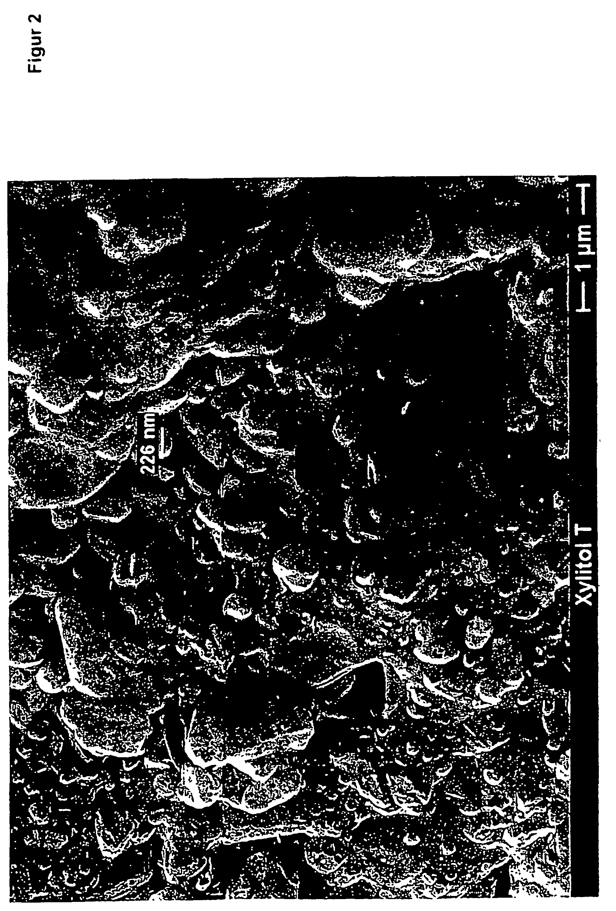

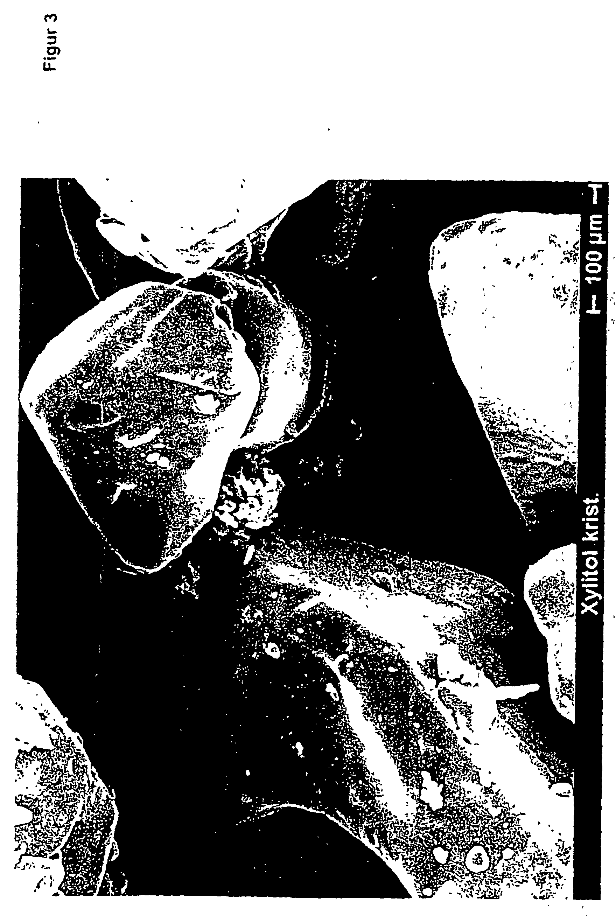

In den Figuren 1 und 2 sind REM-Aufnahmen der Xylitolpulver gemäß Beispiel 1 und in der Figur 3 gemäß dem Vergleichsbeispiel V gezeigt. An den Aufnahmen, insbesondere der Figuren 2 und 3, ist deutlich zu erkennen, dass das erfindungsgemäße Xylitolpulver gemäß Beispiel 1 eine ganz spezielle Partikelmorphologie mit einer extremen Oberflächenzerklüftung und einer hohen Rauhigkeit besitzt und das Vergleichsprodukt V eine glatte, runde Oberfläche hat.

C: Herstellung von Tabletten und deren Eigenschaften

Aus den Xylitolpulvern gemäß den Beispielen 1 und 2, die zur Verringerung des Wassergehaltes auf ca. 0,1 % noch zusätzlich getrocknet wurden, sowie dem Vergleichsbeispiel V wurden unter folgenden Bedingungen Tabletten hergestellt:

Zur Tablettierung wurden jeweils 500 g Xylitolpulver unter Zusatz von 0,5 % Magnesiumstearat als Gleitmittel im Rhönradmischer mit Erweka-Antrieb während einer Zeitdauer von 2 Minuten gemischt.

Aus den Mischungen wurden mittels einer Presse (Gerät: Korsch Pharmapress PK 100 DMS) jeweils ca. 100 Tabletten mit mittleren Tablettengewichten von ca. 600 mg bei einem Pressdruck von ca. 15 kN hergestellt.

An jeweils 20 Tabletten wurden die Bruchfestigkeit (Gerät: Erweka BFT 30 - Einzelmessung der Bruchkraft) und die Tablettenhöhe (Gerät: Erweka BTT 30 - Einzelmessung der Tablettenhöhe) gemessen und aus den Messwerten der Mittelwert gebildet.

Als weitere Tabletteneigenschaft wurde an 20 Tabletten der Abrieb entsprechend Ph. Eur., indem eine Rückwägung durchgeführt wurde (Gerät: Erweka Friabilitätstester), bestimmt:

Außerdem wurde die Biegefestigkeit rechnerisch nach folgender Gleichung ermittelt:

$$\alpha = N/h^2,$$

wobei

N die Bruchfestigkeit und h die Tablettenhohe bedeuten.

Die Tabletteneigenschaften wurden a) unmittelbar nach der Herstellung der Tabletten und b) nach einer Lagerzeit der Tabletten von 24 h bestimmt.

Die ermittelten Eigenschaftswerte der Tabletten sind in den nachfolgenden Tabellen angegeben.

a) Ergebnisse unmittelbar nach der Herstellung der Tabletten

| Beispiele | Bruchfestigkeit N | Biegefestigkeit N/mm$^2$ | Tablettenhöhe mm | Abrieb % |
|-----------|-------------------|--------------------------|------------------|----------|
| 1 | 132 | 8,56 | 3,925 | 2 |
| 2 | 142 | 9,33 | 3,901 | 1,5 |
| V | 15,7 | 1,075 | 3,823 | 100 |

b) Ergebnisse nach einer Lagerzeit der Tabletten von 24 h

| Beispiele | Bruchfestigkeit N | Biegefestigkeit N/mm$^2$ | Tablettenhöhe mm | Abrieb % |
|-----------|-------------------|--------------------------|------------------|----------|
| 1 | 167,5 | 10,91 | 3,918 | 1 |
| 2 | 170,1 | 11,14 | 3,908 | 1 |
| V | 19,4 | 1,28 | 3,889 | 100 |

In einem weiteren Versuch wurden noch Tabletten aus dem Xylitolpulver gemäß dem Vergleichsbeispiel V unter Anwendung einer erhöhten Presskraft von 32 kN hergestellt.

An diesen Tabletten (jeweils 20 Stuck) wurden als Mittelwerte die Tablettenhärte (Bruchfestigkeit) bestimmt:

a) unmittelbar nach der Herstellung der Tabletten betrug diese 34 N und
b) nach einer Lagerzeit von 24 h der Tabletten 42 N.

Diese Tabletten konnten hinsichtlich der Tablettenhärte nicht die gewünschten Anforderungen von mindestens 140 kN erfüllen. Das Xylitolpulver gemäß dem Vergleichsbeispiel V ist somit für eine Herstellung von Tabletten ungeeignet.

Sensorische Beurteilung der hergestellten Tabletten:

Ein Panel mit 10 Testpersonen übernahm die sensorische Beurteilung. Testergebnisse:

a) Tabletten gemäß den Beispielen 1 und 2
     Die Tabletten verfügen über ein ausgezeichnetes Mundgefühl. Die Oberfläche ist glatt. Dieses Mundgefühlgefühl bleibt auch während des gesamten Lutschvorganges erhalten.
b) Tabletten gemäß dem Vergleichsbeispiel V

Die Tabletten zerfallen sofort im Mund.

**Patentansprüche**

1. Sprühgetrockneter Xylitol mit einem Xylitolgehalt von mindestens 98 Gew.-%, bestehend aus Xylitolpartikeln, **dadurch gekennzeichnet, dass** die einzelnen Xylitolpartikel eine unregelmäßige, zerklüftete Oberfläche besitzen und als rieselfähiges Rein-Xylitolpulver mit einem Wassergehalt von ca. 0,2 bis 1 % und mit einem Kornspektrum von ca. 0,1 bis 0,8 mm innerhalb des Sprühturmes entstehen, wobei das am Sprühturmaustritt anfallende Rein-Xylitolpulver eine Schüttdichte, bestimmt nach DIN ISO 3944, von kleiner als 0,65 kg/l, eine Lösezeit von kleiner als 10 s und eine Rieselzeit von kleiner als 12 s/100 ml oder einen Schüttwinkel von kleiner als 33° besitzt.

2. Sprühgetrockneter Xylitol nach Anspruch 1, **dadurch gekennzeichnet, dass** die Schüttdichte 0,59 bis 0,63 kg/l, die Lösezeit 6 bis 8 s und die Rieselzeit 9 bis 11 s/100 ml oder der Schüttwinkel 27 bis 32° betragen.

3. Sprühgetrockneter Xylitol nach einem der Ansprüche 1 oder 2, **dadurch gekennzeichnet, dass** das Kornspektrum von 0,1 bis 0,315 mm größer als 50 % ist.

4. Verfahren zu Herstellung von pulverförmigem Xylitol nach einem der Ansprüche 1 bis 3, ausgehend von einer Xylitollösung mit einem Feststoffgehalt von 40 bis 80 Gew.-% durch Sprühtrocknung, **dadurch gekennzeichnet, dass** die auf eine Temperatur von 40 bis 95 °C erwärmte Xylitollösung in definierter Menge mittels eines Rotationszerstäubers im Sprühturm versprüht wird unter gleichzeitiger Zuführung von im Kreislauf geführtem pulverförmigen Xylitol in 1 bis 3facher Menge, bezogen auf die eingesetzte Menge an Xylitollösung, und Einblasen von heißer Luft mit einer Temperatur von 110 bis 160 °C in einer Menge von 10 bis 16 $Nm^3$/kg Xylitollösung, wobei die Temperatur der eingeblasenen heißen Luft so geregelt wird, dass sich am Austritt des Sprühturmes eine nahezu konstante Lufttemperatur von 55 bis 60 °C einstellt, und am Sprühturmaustritt bereits rieselfähiges, direkt weiterverarbeitbares Pulver mit einem Kornspektrum von ca. 0,1 bis 0,8 mm mit einer unregelmäßig zerklüfteten Oberfläche und einem Wassergehalt von ca. 0,2 bis 1 % anfällt.

5. Verfahren nach Anspruch 4, **dadurch gekennzeichnet, dass** als Xylitollösung das unmittelbar nach der Hydrierung von Xylose anfallende Hydrierprodukt eingesetzt wird.

6. Verfahren nach einem der Ansprüche 4 oder 5, **dadurch gekennzeichnet, dass** das am Austritt des Sprühturmes anfallende Xylitolpulver auf einem nachgeschalteten Fließbett mittels Warmluft bis zu einem Wassergehalt von kleiner als 0,2 % getrocknet und abschließend mittels entfeuchteter Kaltluft auf eine Temperatur von 40 bis 20 °C abgekühlt wird.

7. Verfahren nach einem der Ansprüche 4 bis 6, **dadurch gekennzeichnet, dass** der Rotationszerstäuber mit einer Drehzahl von 5500 bis 15000 U/min betrieben wird.

8. Verwendung von Xylitolpulver nach einem der Ansprüche 1 bis 3 zur Herstellung von bindemittelfreien Komprimaten bei einem Pressdruck von ca. 10 bis 15 kN mit einer Bruchfestigkeit von mindestens 140 N und einer Biegefestigkeit von mindestens 10 N/mm$^2$, bestimmt nach einer Mindestlagerzeit von 24 h.

9. Verwendung von Xylitolpulver nach einem der Ansprüche 1 bis 3 zur Herstellung von Kaugummi mit antikariogener Wirkung.

10. Bindemittelfreie Komprimate, bestehend aus Xylitolpulver mit einem Xylitolgehalt von mindestens 98 Gew.-% sowie einem Kornspektrum von ca. 0,1 bis 0,8 mm, einem Wassergehalt von < 0,5 %, einer Schüttdichte, bestimmt nach DIN ISO 3944, von kleiner als 0,65 kg/l, einer Lösezeit von kleiner als 10 s und eine Rieselzeit von kleiner als 12 s/100 ml oder einem Schüttwinkel von kleiner als 33°, wobei die einzelnen Xylitolpartikel eine unregelmäßig zerklüftete Oberfläche besitzen.

11. Bindemittelfreie Komprimate nach Anspruch 10, **dadurch gekennzeichnet, dass** diese mindestens einen oder mehrere Zusätze aus der Gruppe pharmazeutische Wirkstoffe, Aromastoffe, Farbstoffe, Vitamine und Spurenelemente enthalten.

12. Bindemittelfreie Komprimate nach einem der Ansprüche 10 oder 11, **dadurch gekennzeichnet, dass** diese in Form von Tabletten vorliegen, die bei einem Pressdruck von ca. 10 bis 15 kN hergestellt sind und eine Bruchfestigkeit von mindestens 140 N und eine Biegefestigkeit von mindestens 10 N/mm$^2$, bestimmt nach einer Mindestlagerzeit von 24 h, aufweisen.

**Claims**

1. Spray-dried xylitol with a xylitol content of at least 98 percent by weight, consisting of xylitol particles **characterized in that** each of the xylitol particles have an irregular, fissured surface structure, and they are produced inside the spray tower as flowable pure xylitol powder with a water content of approx. 0.2 to 1 percent and a particle size range from approx. 0.1 mm to 0.8 mm, with the pure xylitol powder at the exit of the spray tower having a bulk density, as determined according to DIN ISO 3944, of less than 0.65 kg/litre, a dissolving time of less than 10 seconds and a rate of flow of less than 12 seconds per 100 ml, and an angle of repose of less than 33°.

2. Spray-dried xylitol according to claim 1 **characterized in that** it exhibits a bulk density between 0.59 kg/litre and 0.63 kg/litre, a solution time between 6 seconds and 8 seconds and a dissolving time between 9 seconds and 11 seconds per 100 ml, or an angle of repose between 27° and 32°.

3. Spray-dried xylitol according to one of the claims 1 or 2 **characterized in that** the particle size range from 0.1 mm to 0.315 mm represents more than 50 percent.

4. Method for producing powdered xylitol according to one of the claims 1 to 3, using a xylitol solution with a solids content between 40 and 80 percent by weight as feedstock for a spray-drying process, **characterized in that** the xylitol solution is heated up to a temperature of 40°C to 95°C and sprayed in discrete quantities by means of a rotary atomizer inside the spray tower, in which at the same time powdered xylitol is cycled and added in one up to three times the quantity of xylitol solution initially put in, and hot air blown in at a temperature between 110°C and 160°C at a rate between 10 $Nm^3$ and 16 $Nm^3$ per kg of xylitol solution, whereby the temperature of the hot air blown in is controlled in such a way as to maintain the air temperature nearly constant at 55°C to 60°C at the exit of the spray tower to obtain already flowable powder with a particle size range from approx. 0.1 mm to 0.8 mm and a water content between approx. 0.2 and 1 percent that can directly be used for further processing.

5. Method according to claim 4 **characterized in that** the immediate product formed by the hydrogenation of xylose, i.e. the xylitol solution is used as feedstock.

6. Method according to one of the claims 4 or 5 **characterized in that** the xylitol powder obtained at the exit of the spray tower is dried in a downstream fluidised bed by means of hot air down to a water content of less than 0.2 percent and subsequently cooled by means of dehumidified cold air down to a temperature between 40°C and 20°C.

7. Method according to one of the claims 4 to 6 **characterized in that** the rotary atomizer is operated at a speed of 5,500 rpm to 15,000 rpm.

8. Use of xylitol powder according to one of the claims 1 to 3 for the manufacture of binders free comprimates produced at a compression pressure between approx. 10 kN and 15 kN, having a breaking strength of at least 140 N and a bending strength of at least 10 $N/mm^2$, determined after a storage time of at least 24 hours.

9. Use of xylitol powder according to one of the claims 1 to 3 for the manufacture of chewing gum that exhibits anti-carious activity.

10. Binders free com primates consisting of xylitol powder with a xylitol content of at least 98 percent by weight and a particle size range from approx. 0.1 mm to 0.8 mm, a water content of less than 0.5 percent, a bulk density, as determined according to DIN ISO 3944, of less than 0.65 kg/litre, a dissolving time of less than 10 seconds and a rate of flow of less than 12 seconds per 100 ml, or an angle of repose of less than 33°, with each of the xylitol particles having an irregular, fissured surface structure.

11. Binders free comprimates according to claim 10 **characterized in that** such comprimates contain at least one or more additives belonging to the group of active pharmaceutical ingredients, flavours, colours, vitamins or trace elements.

12. Binders free comprimates according to one of the claims 10 or 11 **characterized in that** such comprimates come in the form of tablets produced at a compression pressure between approx. 10 kN and 15 kN, having a breaking strength of at least 140 N and a bending strength of at least 10 $N/mm^2$, determined after a storage time of at least 24 hours.

**Revendications**

1. Xylitol séché par pulvérisation présentant une teneur en xylitol d'au moins 98 % du poids, composé de particules de xylitol, **caractérisé en ce que** chaque particule de xylitol présente une surface irrégulière fissurée et est produite à l'intérieur de la tour de pulvérisation sous forme de poudre de xylitol pur coulable présentant une teneur en eau comprise entre env. 0,2 et 1 % ainsi qu'une répartition granulométrique comprise entre env. 0,1 et 0,8 mm, la poudre de xylitol pur produite à la sortie de la tour de pulvérisation présentant une masse volumique apparente conforme à la norme DIN ISO 3944 et inférieure à 0,65 kg/l, une durée de dissolution inférieure à 10 s et une durée d'écoulement inférieure à 12 s/100 ml ou un angle de déversement inférieur à 33°.

2. Xylitol séché par pulvérisation selon la revendication 1, **caractérisé en ce que** la masse volumique apparente est comprise entre 0,59 et 0,63 kg/l, la durée de dissolution entre 6 et 8 s et la durée d'écoulement entre 9 et 11 s/100 ml ou l'angle de déversement entre 27 et 32°.

3. Xylitol séché par pulvérisation selon l'une quelconque des revendications 1 ou 2, **caractérisé en ce que** la répartition granulométrique comprise entre 0,1 et 0,315 mm est supérieure à 50 %.

4. Procédé pour la préparation de xylitol en poudre selon l'une quelconque des revendications 1 à 3, à partir d'une solution de xylitol présentant une teneur à matières solides comprise entre 40 et 80 % du poids par séchage par pulvérisation, **caractérisé en ce que** la solution de xylitol portée à une température comprise entre 40 et 95°C est pulvérisée dans la tour de pulvérisation, en quantité définie, au moyen d'un atomiseur rotatif en y ajoutant simultanément du xylitol en poudre réutilisé dans une quantité une à trois fois supérieure à la quantité utilisée de solution de xylitol, et en insufflant de l'air chaud à une température comprise entre 110 et 160°C dans une quantité comprise entre 10 et 16 Nm$^3$/kg de solution de xylitol, la température de l'air chaud insufflé étant réglée de sorte qu'une température de l'air presque constante comprise entre 55 et 60°C se règle à la sortie de la tour de pulvérisation, et une poudre déjà coulable, pouvant être directement traitée et présentant une répartition granulométrique comprise entre env. 0,1 et 0,8 mm ainsi qu'une surface irrégulièrement fissurée et une teneur en eau comprise entre env. 0,2 et 1 %, est produite à la sortie de la tour de pulvérisation.

5. Procédé selon la revendication 4, **caractérisé en ce que** le produit hydrogéné produit immédiatement après l'hydrogénation de xylose est utilisé comme solution de xylitol.

6. Procédé selon l'une quelconque des revendications 4 ou 5, **caractérisé en ce que** la poudre de xylitol produite à la sortie de la tour de pulvérisation est séchée sur un lit fluidisé placé en aval avec de l'air chaud jusqu'à obtention d'une teneur en eau inférieure à 0,2 % et est ensuite refroidie avec de l'air froid déshumidifié jusqu'à obtention d'une température comprise entre 40 et 20°C.

7. Procédé selon l'une quelconque des revendications 4 à 6, **caractérisé en ce que** l'atomiseur rotatif est actionné à une vitesse de rotation comprise entre 5 500 et 15 000 tr/min.

8. Utilisation de poudre de xylitol selon l'une quelconque des revendications 1 à 3 pour la préparation de produits comprimés sans liant à une pression de compression comprise entre env. 10 et 15 kN présentant une résistance à la rupture d'au moins 140 N et une résistance à la flexion d'au moins 10 N/mm$^2$, déterminées après une durée de stockage minimale de 24 h.

9. Utilisation de poudre de xylitol selon l'une quelconque des revendications 1 à 3 pour,la préparation de chewing-gum à effet anticariogène.

10. Produits comprimés sans liant, composés de poudre de xylitol présentant une teneur en xylitol d'au moins 98 % en poids ainsi qu'une répartition granulométrique comprise entre env. 0,1 et 0,8 mm, une teneur en eau de < 0,5 %, une masse volumique apparente conforme à la norme DIN ISO 3944 et inférieure à 0,65 kg/l, une durée de dissolution inférieure à 10 s et une durée d'écoulement inférieure à 12 s/100 ml ou un angle de déversement inférieur à 33°, chaque particule de xylitol présentant une surface irrégulièrement fissurée.

11. Produits comprimés sans liant selon la revendication 10, **caractérisés en ce que** ceux-ci contiennent au moins un ou plusieurs additifs Issus du groupe des principes actifs pharmaceutiques, des substances aromatisantes, des substances colorantes, des vitamines et des oligoéléments.

12. Produits comprimés sans liant selon l'une quelconque des revendications 10 ou 11, **caractérisés en ce que** ceux-ci se présentent sous forme de comprimés qui sont préparés à une pression de compression comprise entre env. 10 et 15 kN et qui présentent une résistance à la rupture d'au moins 140 N et une résistance à la flexion d'au moins 10 N/mm$^2$, déterminées après une durée de stockage minimale de 24 h.

**Figur 1**

Xylitol T

⊢ 100 µm ⊤

Figur 2

226 nm

Xylitol T

⊢— 1 μm —⊣

EP 1 424 319 B1

Figur 3